# EUROPEAN PATENT APPLICATION

(11) **EP 4 670 632 A1**
(43) Date of publication of application: **31.12.2025**
(21) Application number: 25175661.5
(22) Date of filing: 12.05.2025
(51) Int. Cl.: A61B 5/346, A61B 5/00

(54) **SYSTEM AND METHOD FOR EXTRACTING, USING AN ARTIFICIAL INTELLIGENCE MODEL, CRITERIA FOR DETERMINING A DIAGNOSIS BY A RULE-BASED ECG ANALYSIS MODEL**

(30) Priority: 05.06.2024 GR 20240100414; 06.06.2024 US 202463656871 P
(71) Applicant: GE Precision Healthcare LLC, Waukesha, WI 53188 (US)
(72) Inventor: KARGIANTOULAKIS, Emmanouil, Waukesha, 53188 (US)
(74) Representative: Kilburn & Strode LLP

(57) **Abstract**

A system and method for determining, by a rule-based ECG analysis model, a diagnosis of an ECG using criteria extracted by an AI model are provided. An ECG may be received by the rule-based ECG analysis model. Features of the ECG may be determined by the rule-based ECG analysis model. The diagnosis may be determined by the rule-based ECG analysis model using the features of the ECG and the criteria extracted by the AI model. The diagnosis may be transmitted.

## Description

### CROSS- REFERENCE TO RELATED APPLICATION(S)

This application claims priority to Greek Patent Application No. 20240100414, filed on June 5, 2024 in the Greek Patent Office, which is incorporated by reference herein in its entirety.

### TECHNICAL FIELD

The present disclosure relates to a rule-based ECG analysis model that determines a diagnosis of an ECG using criteria. More specifically, the present disclosure relates to extracting, using an artificial intelligence (AI) model, criteria for determining a diagnosis by a rule-based ECG analysis model.

### BACKGROUND

An ECG is a graphic representation of electrical activity of the heart, and is generally represented as a waveform. A rule-based ECG analysis model may receive an ECG, determine a set of features of the ECG, compare the set of features to respective criteria of a set of rules, determine a diagnosis, and generate a diagnostic interpretation result of the ECG. A physician may review the ECG and/or the diagnostic interpretation result to assess the cardiac activity of a patient.

A rule of the rule-based ECG analysis model may correspond to a particular diagnosis. Further, a rule may include various criteria that, if satisfied, cause the rule-based ECG analysis model to determine the particular diagnosis. A criterion may correspond to a particular feature of the ECG, and may include a relevant threshold. As an example, the rule-based ECG analysis model may use a criterion corresponding to a feature of an R-prime area in lead aVL being less than or equal to a threshold of 3564.5. The rule-based ECG analysis model may determine a particular diagnosis of left bundle branch block (LBBB) based on an R-prime area in lead aVL of an ECG being less than or equal to the threshold of 3564.5. In this way, the rule-based ECG analysis model may use fully-interpretable criteria when determining diagnoses. The criteria may be fully-interpretable in that a reviewing physician may ascertain the particular feature and the particular threshold that resulted in a particular diagnosis. In other words, a physician may understand the decision-making of the rule-based ECG analysis model.

In some cases, a subject-matter expert may attempt to extract rules and/or criteria for implementation by the rule-based ECG analysis model. The process of extracting the rules and/or criteria may be arduous, inefficient, and/or impossible. Moreover, the extracted rules and/or criteria may be inaccurate, incomplete, and/or spurious.

An AI model may be used for ECG interpretation. For example, an AI model may be trained on training data including ECGs and corresponding diagnoses. The AI model may receive an ECG, generate a diagnosis, and output the diagnosis. Although AI models may be efficient and accurate, the decision-making of the AI models might not be interpretable. That is, a reviewing physician might not be able to ascertain how the AI model determined a diagnosis and/or might not be able to ascertain the underlying features on which the AI model determined the diagnosis. Further, the AI model might be prone to bias caused by inaccuracies in the training data, inaccuracies in the training process, or the like. Accordingly, the implementation of AI models for ECG interpretation in clinical settings might be difficult or impossible.

### SUMMARY

This summary introduces concepts that are described in more detail in the detailed description. It should not be used to identify essential features of the claimed subject matter, nor to limit the scope of the claimed subject matter.

According to an aspect, a method may include receiving, by a rule-based electrocardiogram (ECG) model, an ECG; determining, by the rule-based ECG model, features of the ECG; determining, by the rule-based ECG model, a diagnosis using the features of the ECG and criteria extracted by an artificial intelligence (AI) model; and transmitting the diagnosis.

According to an aspect, a device may include a memory configured to store instructions; and one or more processors configured to: receive, by a rule-based electrocardiogram (ECG) model of the device, an ECG; determine, by the rule-based ECG model of the device, features of the ECG; determine, by the rule-based ECG model of the device, a diagnosis using the features of the ECG and criteria extracted by an artificial intelligence (AI) model; and transmit the diagnosis.

According to an aspect, a non-transitory computer-readable medium may store instructions that, when executed by one or more processors of a device, cause the one or more processors to: receive, by a rule-based electrocardiogram (ECG) model of the device, an ECG; determine, by the rule-based ECG model of the device, features of the ECG; determine, by the rule-based ECG model of the device, a diagnosis using the features of the ECG and criteria extracted by an artificial intelligence (AI) model; and transmit the diagnosis.

### BRIEF DESCRIPTION OF DRAWINGS

FIG. 1 is a diagram of an example system for extracting, using an AI model, criteria for determining a diagnosis by a rule-based ECG analysis model.
FIG. 2 is a diagram of example components of a device of the example system shown in FIG. 1.
FIG. 3 is a flowchart of an example process for extracting, using an AI model, criteria for determining a diagnosis by a rule-based ECG analysis model.
FIG. 4 is a diagram of an example process for extracting, using an AI model, criteria for determining a diagnosis by a rule-based ECG analysis model, and updating the rule-based ECG analysis model using the criteria.
FIGS. 5A and 5B are diagrams of an example AI model for extracting criteria for determining a diagnosis by a rule-based ECG analysis model.
FIG. 6 is a diagram of example criteria for determining a diagnosis by a rule-based ECG analysis model.
FIG. 7 is a diagram of an example graph of an F1 score and an error score threshold.
FIG. 8 is a diagram of an example graph of a precision and a sensitivity of the rule-based ECG analysis model.
FIG. 9 is a flowchart of an example process for determining a diagnosis using features of an ECG and criteria, extracted by an AI model, for determining the diagnosis.
FIG. 10 is a diagram of an example process for determining a diagnosis using features of an ECG and criteria, extracted by an AI model, for determining the diagnosis.

### DETAILED DESCRIPTION

As addressed above, the process of extracting rules and/or criteria for determining a diagnosis by a rule-based ECG analysis model may be arduous, inefficient, and/or impossible, and the extracted rules and/or criteria may be inaccurate, incomplete, and/or spurious. Further, as addressed above, the implementation of AI models for ECG interpretation may be efficient and accurate. However, the decision-making of the AI models might not be interpretable.

Some embodiments of the present disclosure provide a system and method for extracting, using an AI model, interpretable criteria for determining a diagnosis by a rule-based ECG analysis model. Some embodiments herein may improve the performance of rule-based ECG analysis models, which may reduce the need to correct the outputs of the rule-based ECG analysis models, may improve treatment decisions by physicians, may improve outcomes for patients, or the like. Further, the extracted criteria may be fully-interpretable, which may reduce the potential for bias, may reduce uncertainty and confusion in the decision-making, and may improve the ability to integrate the extracted rules and criteria in clinical workflows.

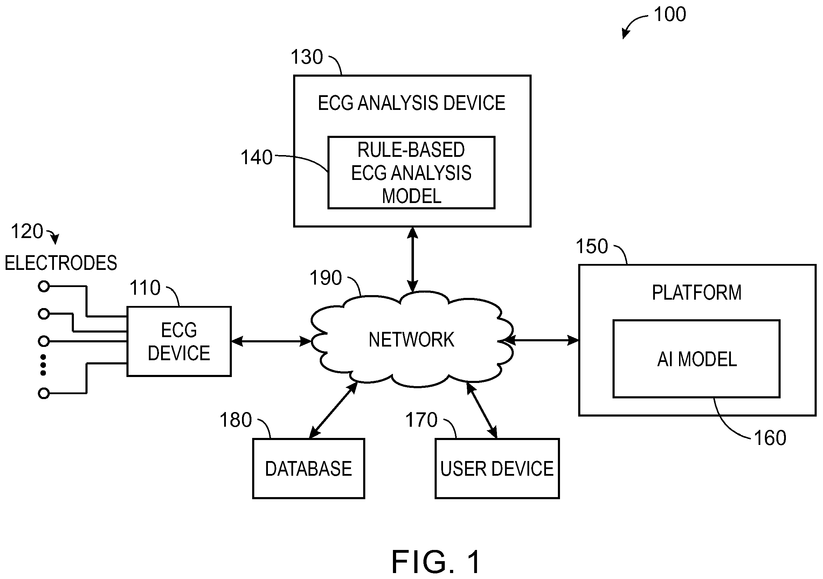

FIG. 1 is a diagram of an example system 100 for extracting, using an AI model, interpretable criteria for determining a diagnosis by a rule-based ECG analysis model. As shown in FIG. 1, the system 100 may include an ECG device 110, electrodes 120, an ECG analysis device 130, a rule-based ECG analysis model 140, a platform 150, an AI model 160, a user device 170, a database 180, and a network 190.

The ECG device 110 may be configured to generate an ECG of a patient. For example, the ECG device 110 may be a stand-alone ECG device, a portable ECG device, a multi vital sign monitoring device, or the like. The ECG device 110 may receive cardiac electrical signals via the electrodes 120, and generate the ECG based on the cardiac electrical signals. The ECG may be a single-lead ECG, 3-lead ECG, a 5-lead ECG, a 6-lead ECG, a 12-lead ECG, or the like. The ECG device 110 may include any number of electrodes 120. For example, the ECG device 110 may include electrodes for generating a 12-lead ECG. In this case, the leads may include leads I, II, III, aVF, aVR, aVL, V1, V2, V3, V4, V5, and V6. The ECG device 110 may be configured to use a subset of the standard 12 leads, or alternatively an ECG using non-standard lead placements (e.g., such as Holter lead placements) or synthesized ECG leads using the actual acquired lead set (e.g. GE HealthCare's 12RL algorithm used to synthesize a 12 lead ECG from a reduced lead set).

The ECG analysis device 130 may be configured to receive the ECG from the ECG device 110, determine a set of features of the ECG using the rule-based ECG analysis model 140, compare the set of features to respective criteria of a set of rules using the rule-based ECG analysis model 140, determine a diagnosis using the rule-based ECG analysis model 140, generate a diagnostic interpretation result of the ECG using the rule-based ECG analysis model 140, and transmit the diagnostic interpretation result to another device, to a display, or the like. For example, the ECG analysis device 130 may be a server, a cloud computing system, a standalone medical device, or the like.

The rule-based ECG analysis model 140 may be configured to receive the ECG from the ECG device 110, determine a set of features of the ECG, compare the set of features to respective criteria of a set of rules, determine a diagnosis, and generate a diagnostic interpretation result of the ECG. For example, the rule-based ECG analysis model 140 may be the Marquette^{™} 12SL ECG analysis program, or the like.

The platform 150 may be configured to extract, using the AI model 160, interpretable criteria for determining a diagnosis by the rule-based ECG analysis model 140. For example, the platform 150 may be a server, a cloud computing system, or the like.

The AI model 160 may be configured to extract interpretable criteria for determining a diagnosis by the rule-based ECG analysis model 140. For example, the AI model 160 may be a decision tree (e.g., a classification tree, a regression tree, or the like), a linear regression model, a neural network (e.g., a deep neural network (DNN), a convolutional neural network (CNN), a recurrent neural network (RNN), or the like), a logistic regression model, a support vector machine, or the like.

The user device 170 may be configured to receive the criteria for determining a diagnosis by the rule-based ECG analysis model 140, and provide the criteria for determining a diagnosis by the rule-based ECG analysis model 140 for display. For example, the user device 170 may be a smartphone, a laptop computer, a desktop computer, a wearable device, a medical device, or the like.

The database 180 may be configured to store an ECG, a set of features of the ECG, a diagnosis of the ECG, a diagnostic interpretation result of the ECG, modification information, patient information associated with the ECG, a classification target of the ECG, or the like. Further, the database 180 may be configured to store criteria for determining a diagnosis by the rule-based ECG analysis model 140. For example, the database 180 may be a hierarchical database, a network database, a relational database, or the like.

The network 190 may be configured to permit communication between the devices of the system 100. For example, the network 190 may be a cellular network (e.g., a fifth generation (5G) network, a long-term evolution (LTE) network, a third generation (3G) network, a code division multiple access (CDMA) network, etc.), a public land mobile network (PLMN), a local area network (LAN), a wide area network (WAN), a metropolitan area network (MAN), a telephone network (e.g., the Public Switched Telephone Network (PSTN)), a private network, an ad hoc network, an intranet, the Internet, a fiber optic-based network, or the like, and/or a combination of these or other types of networks.

The number and arrangement of the devices of the system 100 shown in FIG. 1 are provided as an example. In practice, the system 100 may include additional devices, fewer devices, different devices, or differently arranged devices than those shown in FIG. 1. Additionally, or alternatively, a set of devices (e.g., one or more devices) of the system 100 may perform one or more functions described as being performed by another set of devices of the system 100.

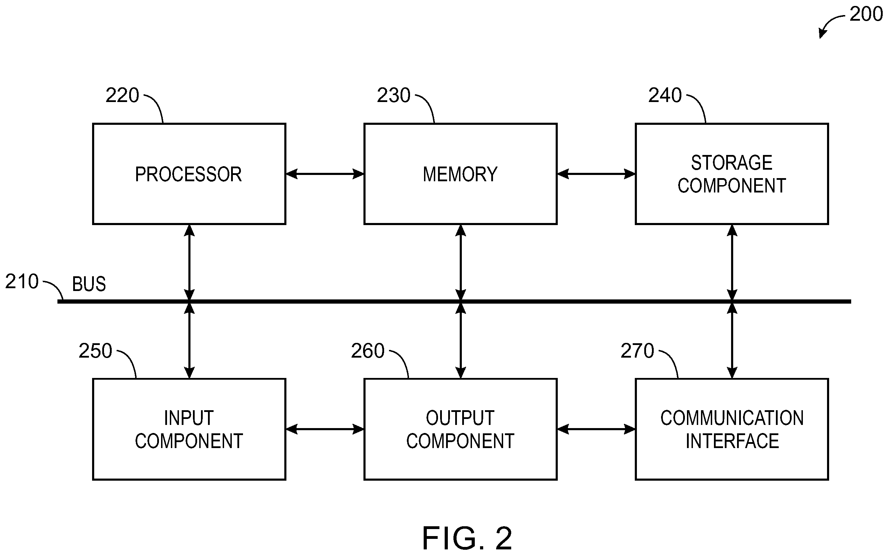

FIG. 2 is a diagram of example components of a device 200 of the example system shown in FIG. 1. The device 200 may correspond to the ECG device 110, the ECG analysis device 130, the platform 150, the user device 170, and/or the database 180. As shown in FIG. 2, the device 200 may include a bus 210, a processor 220, a memory 230, a storage component 240, an input component 250, an output component 260, and a communication interface 270.

The bus 210 includes a component that permits communication among the components of the device 200. The processor 220 may be implemented in hardware, firmware, or a combination of hardware and software. The processor 220 may be a central processing unit (CPU), a graphics processing unit (GPU), an accelerated processing unit (APU), a microprocessor, a microcontroller, a digital signal processor (DSP), a field-programmable gate array (FPGA), an application-specific integrated circuit (ASIC), or another type of processing component. The processor 220 may include one or more processors capable of being programmed to perform a function. The processor 220 may include one or more processors 220 configured to perform the operations described herein. For example, a single processor 220 may be configured to perform all of the operations described herein. Alternatively, multiple processors 220, collectively, may be configured to perform all of the operations described herein, and each of the multiple processors 220 may be configured to perform a subset of the operations described herein. For example, a first processor 220 may perform a first subset of the operations described herein, a second processor 220 may be configured to perform a second subset of the operations described herein, etc.

The memory 230 may include a random access memory (RAM), a read only memory (ROM), and/or another type of dynamic or static storage device (e.g., a flash memory, a magnetic memory, and/or an optical memory) that stores information and/or instructions for use by the processor 220.

The storage component 240 may store information and/or software related to the operation and use of the device 200. For example, the storage component 240 may include a hard disk (e.g., a magnetic disk, an optical disk, a magneto-optic disk, and/or a solid state disk), a compact disc (CD), a digital versatile disc (DVD), a floppy disk, a cartridge, a magnetic tape, and/or another type of non-transitory computer-readable medium, along with a corresponding drive.

The input component 250 may include a component that permits the device 200 to receive information, such as via user input (e.g., a touch screen display, a keyboard, a keypad, a mouse, a button, a switch, a camera, and/or a microphone for receiving the reference audio input and/or visual input). Additionally, or alternatively, the input component 250 may include a sensor for sensing information (e.g., a global positioning system (GPS) component, an accelerometer, a gyroscope, and/or an actuator). The output component 260 may include a component that provides output information from the device 200 (e.g., a display, a speaker for outputting sound at the output sound level, and/or one or more light-emitting diodes (LEDs)).

The communication interface 270 may include a transceiver-like component (e.g., a transceiver and/or a separate receiver and transmitter) that enables the device 200 to communicate with other devices, such as via a wired connection, a wireless connection, or a combination of wired and wireless connections. The communication interface 270 may permit the device 200 to receive information from another device and/or transmit information to another device. For example, the communication interface 270 may include an Ethernet interface, an optical interface, a coaxial interface, an infrared interface, a radio frequency (RF) interface, a universal serial bus (USB) interface, a Wi-Fi interface, a cellular network interface, or the like.

The device 200 may perform one or more processes described herein. The device 200 may perform these processes based on the processor 220 executing software instructions stored by a non-transitory computer-readable medium, such as the memory 230 and/or the storage component 240. A computer-readable medium may be defined herein as a non-transitory memory device. A memory device may include memory space within a single physical storage device or memory space spread across multiple physical storage devices.

The software instructions may be read into the memory 230 and/or the storage component 240 from another computer-readable medium or from another device via the communication interface 270. When executed, the software instructions stored in the memory 230 and/or the storage component 240 may cause the processor 220 to perform one or more processes described herein. Additionally, or alternatively, hardwired circuitry may be used in place of or in combination with software instructions to perform one or more processes described herein. Thus, implementations described herein are not limited to any specific combination of hardware circuitry and software.

The number and arrangement of the components shown in FIG. 2 are provided as an example. In practice, the device 200 may include additional components, fewer components, different components, or differently arranged components than those shown in FIG. 2. Additionally, or alternatively, a set of components (e.g., one or more components) of the device 200 may perform one or more functions described as being performed by another set of components of the device 200.

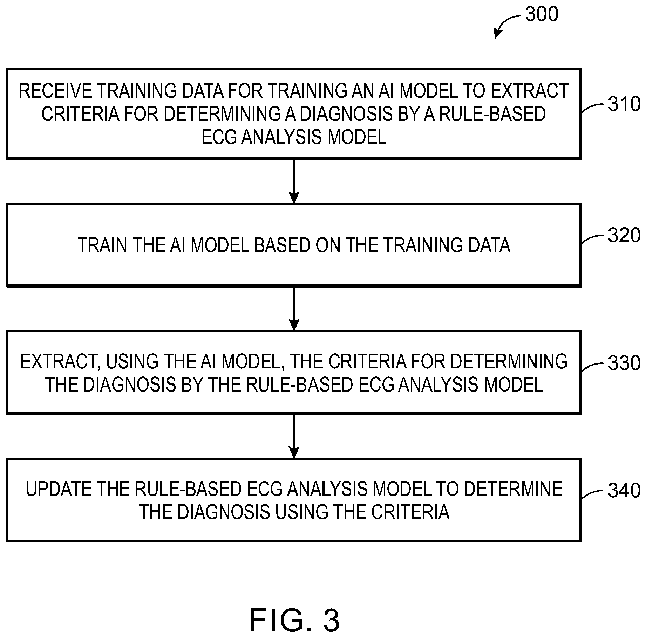

FIG. 3 is a flowchart of an example process 300 for extracting, using an AI model, criteria for determining a diagnosis by a rule-based ECG analysis model.

As shown in FIG. 3, the process 300 may include receiving training data for training an AI model to extract interpretable criteria for determining a diagnosis by a rule-based ECG analysis model (operation 310). For example, the platform 150 may receive training data for training the AI model 160 to extract criteria for determining a diagnosis by the rule-based ECG analysis model 140.

According to an embodiment, the training data may include an ECG. For example, the ECG may be a waveform acquired via the ECG device 110. Additionally, or alternatively, the training data may include a set of features of the ECG. For example, the set of features may include an amplitude of a P wave, a duration of the P wave, an amplitude of a Q wave, a duration of the Q wave, an amplitude of an R wave, a duration of the R wave, a PR interval, an amplitude of an S wave, a duration of the S wave, a duration of the QRS complex, an amplitude of a T wave, a duration of the T wave, a QT interval, or the like. Additionally, or alternatively, the set of features may be defined by the data from a single lead (e.g., single lead specific P wave amplitude or P wave duration), or by data from multiple leads (e.g., global QT duration from earliest Q onset to latest T offset across all leads, or QT dispersion, which is a difference between shortest and longest single lead QT interval measurement across all leads), or by data from spatial relationships across multiple leads (e.g., spatial QRS-T angle or ventricular gradient).

Additionally, or alternatively, the training data may include a diagnosis of the ECG. For example, the diagnosis may include atrial-paced rhythm, ventricular-paced rhythm, atrial flutter, ectopic atrial tachycardia, sinus bradycardia, sinus tachycardia, junctional bradycardia, atrial fibrillation, left bundle branch block, septal infarct, or the like. Additionally, or alternatively, the training data may include a diagnostic interpretation result of the ECG. For example, the diagnostic interpretation result may be an output of the rule-based ECG analysis model 140. Additionally, or alternatively, the diagnostic interpretation result may be an interpretation result of the ECG determined by a physician. Additionally, or alternatively, the diagnostic interpretation result may be a diagnosis by physician using the ECG alone, or alternatively using another source of clinical information (e.g., high sensitivity troponin levels or cardiac echo measurements), or a combination of ECG and non-ECG clinical information.

Additionally, or alternatively, the training data may include modification information that identifies whether the diagnostic interpretation result was modified. For example, the modification information may identify whether a diagnosis of the diagnostic interpretation result determined by the rule-based ECG analysis model 140 was accepted, removed, or replaced. As another example, the modification information may identify whether a diagnosis that was not determined by the rule-based ECG analysis model 140 was added. The modification information may identify a replacement diagnosis in the event that the diagnosis was replaced. According to an embodiment, the platform 150 may determine the modification information based on a diagnostic interpretation result determined by the rule-based ECG analysis model 140 and a final diagnostic interpretation result determined by a physician after the physician reviews the diagnostic interpretation result determined by the rule-based ECG analysis model 140. For example, the platform 150 may compare the diagnostic interpretation result determined by the rule-based ECG analysis model 140 and the final diagnostic interpretation result determined by a physician, and determine the modification information based on the comparison.

Additionally, or alternatively, the training data may include patient information associated with the ECG. For example, the patient information may identify whether a specific diagnosis was present in a previous diagnostic interpretation result of the patient, identify whether a physician had previously modified a diagnosis interpretation result, identify demographic information of the patient, identify health conditions of the patient, identify prescriptions of the patient, identify previous procedures of the patient, identify previous diagnoses of the patient, or the like.

Additionally, or alternatively, the training data may include a classification target of the ECG. For example, the classification target may be a diagnosis of the ECG, a diagnostic interpretation result of the ECG, modification information of the ECG, or the like.

As further shown in FIG. 3, the process 300 may include training the AI model based on the training data (operation 320). For example, the platform 150 may train the AI model 160 based on the training data. Alternatively, a system or device other than the platform 150 may be used to generate and/or train the AI model 160. For example, a system or device may include instructions for generating the AI model 160, and/or instructions for training the AI model 160. The system or device may provide a resulting trained AI model 160 to the platform 150 for use.

According to an embodiment, the AI model 160 may include a training phase, a deployment phase, and a monitoring phase. In the training phase, the platform 150 may receive and process training data to generate the trained AI model 160 for extracting the criteria for determining a diagnosis by the rule-based ECG analysis model 140. The training data may include a plurality of training datasets respectively including one or more of an ECG, a set of features of the ECG, a diagnosis of the ECG, a diagnostic interpretation result of the ECG, modification information, patient information associated with the ECG, a classification target of the ECG, or the like. Each training dataset of the plurality of training datasets may be associated with a particular ECG and a particular diagnostic interpretation result.

The training data may be generated, received, or otherwise obtained from internal and/or external resources. For example, the training data may be generated, received, or otherwise obtained from the ECG device 110, the ECG analysis device 130, the user device 170, and/or the database 180.

Generally, the AI model 160 may include a set of variables (e.g., nodes, neurons, filters, or the like) that are tuned (e.g., weighted, biased, or the like) to different values via the application of the training data. According to an embodiment, the training process may employ supervised, unsupervised, semi-supervised, and/or reinforcement learning processes to train the AI model 160. According to an embodiment, a portion of the training data may be withheld during training and/or used to validate the trained AI model 160.

For supervised learning processes, the training data may include labels or scores that may facilitate the training process by providing a ground truth. For example, the labels or scores may indicate a classification target. Training may proceed by feeding a training dataset into the AI model 160. The AI model 160 may have variables set at initialized values (e.g., at random, based on Gaussian noise, based on pre-trained values, or the like). The AI model 160 may generate an output. The output may be compared with the corresponding label or score (e.g., the ground truth), which may then be back-propagated through the AI model 160 to adjust the values of the variables. This process may be repeated for a plurality of samples at least until a determined loss or error is below a predefined threshold. According to an embodiment, some of the training data may be withheld and used to further validate or test the trained AI model 160.

For unsupervised learning processes, the training data may not include pre-assigned labels or scores to aid the learning process. Instead, unsupervised learning processes may include clustering, classification, or the like, to identify naturally occurring patterns in the training data. As an example, training data may be clustered into groups based on identified similarities and/or patterns. K-means clustering or K-Nearest Neighbors may also be used, which may be supervised or unsupervised. Combinations of K-Nearest Neighbors and an unsupervised cluster technique may also be used. For semi-supervised learning, a combination of training data with pre-assigned labels or scores and training data without pre-assigned labels or scores may be used to train the AI model 160.

When reinforcement learning is employed, an agent (e.g., an algorithm) may be trained to make a decision regarding whether a diagnostic interpretation should be modified from the training data through trial and error. For example, based on making a decision, the agent may then receive feedback (e.g., a positive reward if the prediction was above a predetermined threshold), adjust its next decision to maximize the reward, and repeat until a loss function is optimized.

After being trained, the trained AI model 160 may be stored and subsequently applied by the platform 150 during the deployment phase. For example, during the deployment phase, the trained AI model 160 executed by the platform 150 may receive input data, and generate output data. During a monitoring phase, monitoring data may be analyzed along with the output data and input data to determine an accuracy of the trained AI model 160. According to an embodiment, based on the analysis, the platform 150 may return to the training phase, where values of one or more variables of the AI model 160 may be adjusted to improve the accuracy of the AI model 160.

According to an embodiment, the AI model 160 may be a decision tree. In this case, the platform 150 may generate the decision tree using a training technique. For example, the training technique may include a random forest technique, a boosted trees technique, a bootstrap technique, a rotation forest technique, or the like. The AI model 160 may include a set of nodes. For example, the set of nodes may include a root node, one or more intermediate nodes, and leaf nodes. The platform 150 may generate the AI model 160 using an attribute selection measure. For example, the attribute selection measure may be information gain, a gain ratio, a Gini index, or the like. The platform 150 may generate the decision tree, and prune the decision tree using a pruning technique. For example, the pruning technique may be cost complexity pruning, reduced error pruning, or the like.

As further shown in FIG. 3, the process 300 may include extracting, using the AI model, the criteria for determining the diagnosis by the rule-based ECG analysis model (operation 330). For example, the platform 150 may extract, using the AI model 160, the criteria for determining the diagnosis by the rule-based ECG analysis model 140.

According to an embodiment, the platform 150 may determine a decision branch of the AI model 160 that corresponds to a particular target classification. For example, the target classification may be a diagnosis of the ECG, a diagnostic interpretation result of the ECG, modification information of the ECG, or the like. The decision branch may include one or more nodes corresponding to respective criteria. For example, the decision branch may include a root node, one or more intermediate nodes, and a leaf node.

According to an embodiment, the platform 150 may determine a decision branch of the AI model 160 that includes one or more nodes associated with an attribute selection measure that satisfies a threshold. For example, the attribute selection measure may be information gain, a gain ratio, a Gini index, or the like. As a particular example, the platform 150 may determine a decision branch that includes a leaf node corresponding to a criterion having a Gini index that is less than a threshold. According to an embodiment, the platform 150 may determine a decision branch based on a metric of the decision path. For example, the metric may be an accuracy, a positive prediction value, a sensitivity, or the like. Additionally, or alternatively, the platform 150 may determine a decision branch based on a performance on generalization to external datasets.

According to an embodiment, the platform 150 may determine particular nodes of the one or more nodes to use for criteria extraction. The platform 150 may determine the particular nodes based on a feature selection measure. The feature selection measure may correspond to an importance of a feature associated with a criterion corresponding to the node. According to an embodiment, the platform 150 may fit another AI model 160 (e.g., a decision tree) to a dataset that is separate from the training dataset. In this way, the platform 150 may reduce the risk of overfitting, and increase the statistical power of the technique.

According to an embodiment, the platform 150 may extract the criteria from the decision branch. For example, the platform 150 may extract the criteria that belong to the decision branch. Each criterion may include a respective feature and a respective threshold.

According to an embodiment, the platform 150 may determine a threshold of a criterion. For example, the platform 150 may determine a threshold based on an optimization technique, based on an input from the user device 170, or the like.

According to an embodiment, the platform 150 may generate a rule including a set of criteria that were extracted. For example, the rule may include criteria from a particular decision branch. Alternatively, the rule may include criteria from different decision branches. The platform 150 may generate the rule using a permutation and/or combination of criteria. According to an embodiment, the platform 150 may determine a metric of the rule. For example, the metric may be an accuracy, a positive prediction value, a sensitivity, or the like. Further, the platform 150 may determine that the rule includes a metric that satisfies a threshold.

According to an embodiment, the platform 150 may generate the rule to include a score threshold. For example, the score threshold may, if satisfied, trigger the rule-based ECG analysis model 140 to determine a diagnosis. According to an embodiment, the platform 150 may determine the score threshold based on a performance metric. For example, the performance metric may be an F-score, a precision, a sensitivity, or the like. Each criterion of the rule may be associated with a particular score. The rule-based ECG analysis model 140 may determine an overall score based on the particular criterion that are satisfied. The rule-based ECG analysis model 140 may compare the overall score to the score threshold, and determine a diagnosis based on comparing the overall score to the score threshold. For example, if the overall score is greater than or equal to the score threshold, then the rule-based ECG analysis model 140 may determine a particular diagnosis. Alternatively, if the overall score is less than the score threshold, then the rule-based ECG analysis model 140 may not determine the particular diagnosis. According to an embodiment, the platform 150 may generate the rule to include one or more mandatory criteria. For example, the rule may include a mandatory criterion that must be satisfied in order for the rule-based ECG analysis model 140 to determine a diagnosis regardless of the overall score. In this way, different decision branches may be explored by demanding that some of the criteria are met rather than being added to a score, and by varying how demanding each criterion is. The different decision branches that should be met before giving a positive classification may be thought of as accessing different subgroups, and may be deployed in parallel. For example, when assessing a diagnosis of atrial fibrillation, the platform 150 may consider two different subgroups (e.g., a first subgroup with many P waves detected and a second subgroup with very few P waves detected). Both subgroups may correctly identify relevant targets, but they operate in different parameter regions.

In some cases, the platform 150 may receive input from the user device 170 corresponding to expert knowledge that is leveraged to fine-tune the score. The expert knowledge may include an examination of all ECGs that are elected by the procedure for editing to assess the effect of the rule-based ECG analysis model 140, which is possible based on the full transparency of the criteria. If the expert knowledge indicates that some criteria are not accurate, or were selected due to bias from errors in annotations or labels, then the platform 150 may remove the criteria. If more control is warranted to avoid spurious decisions, then the platform 150 may demand concomitant validation from complementary or reciprocal leads and features. This control and accessibility is possible based on the interpretability of the criteria, and is unparalleled in other models.

According to an embodiment, the platform 150 may validate the rule based on training data. For example, the platform 150 may receive training data from the database 180, and validate the rule. Based on validating the rule, the platform 150 may update the rule-based ECG analysis model 140 to implement the rule.

As further shown in FIG. 3, the process 300 may include updating the rule-based ECG analysis model to determine the diagnosis using the criteria (operation 340). For example, the platform 150 may update the rule-based ECG analysis model 140 based on the criteria. The platform 150 may update the rule-based ECG analysis model such that the rule-based ECG analysis model 140 may determine diagnoses using the criteria. In this way, the rule-based ECG analysis model 140 may receive ECGs, and determine diagnoses of the ECGs using the criteria extracted by the AI model 160.

Although FIG. 3 depicts particular operations and a particular sequence of operations, it should be understood that other embodiments may include different operations, more operations, fewer operations, or differently arranged operations than as shown in FIG. 3.

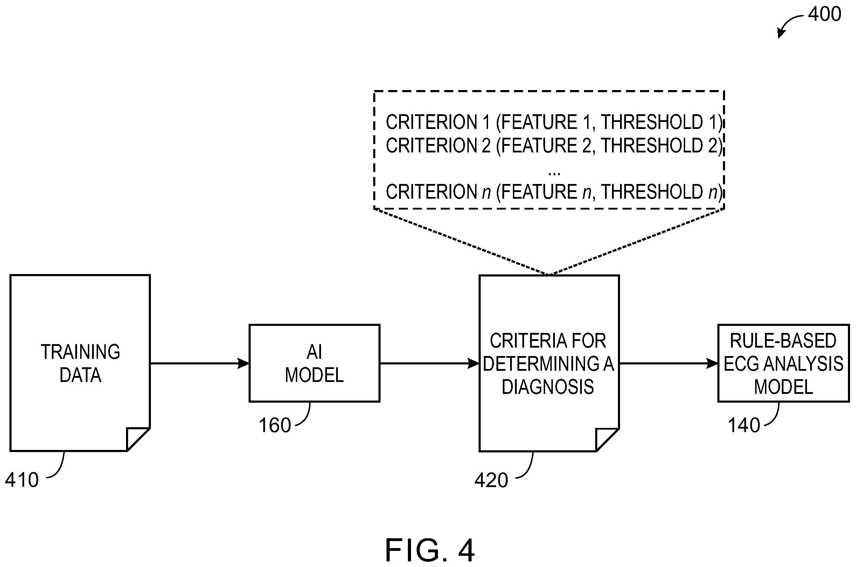

FIG. 4 is a diagram of an example process 400 for extracting, using an AI model, criteria for determining a diagnosis by a rule-based ECG analysis model, and updating the rule-based ECG analysis model using the criteria. As shown in FIG. 4, the AI model 160 may receive training data 410, and extract criteria 420 for determining a diagnosis by the rule-based ECG analysis model 140. For example, as shown, a first criterion ("criterion 1") may include a first feature ("feature 1") of an ECG and a first threshold ("threshold 1"), a second criterion may include a second feature ("feature 2") and a second threshold ("threshold 2"), and an n-th criterion ("criterion *n"*) may include an n-th feature ("feature *n"*) and an n-th threshold ("threshold n"). The rule-based ECG analysis model 140 may receive the criteria, and use the criteria for determining a diagnosis of an ECG.

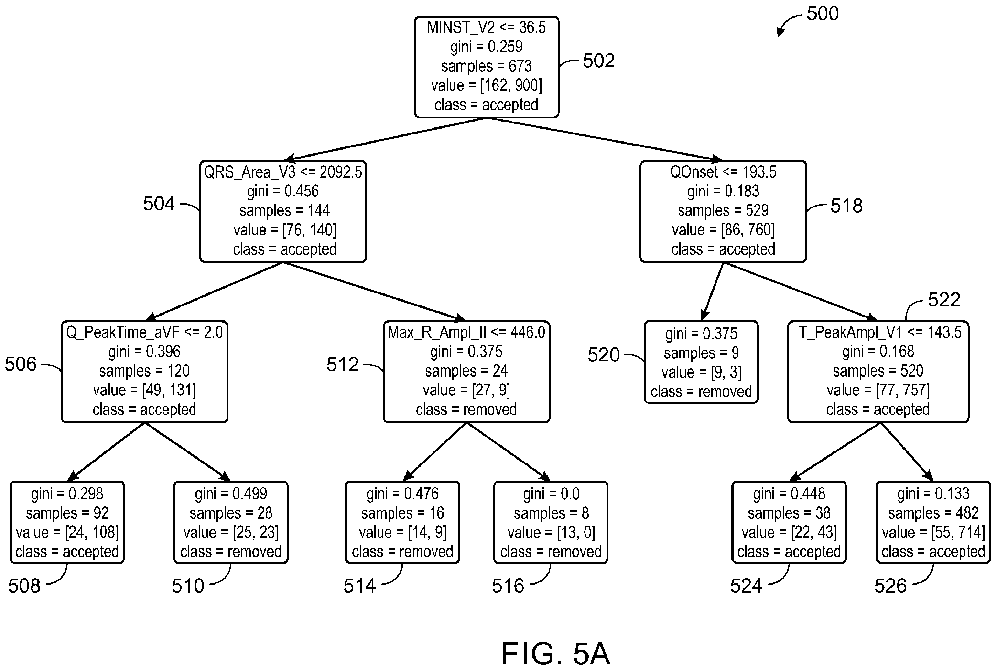

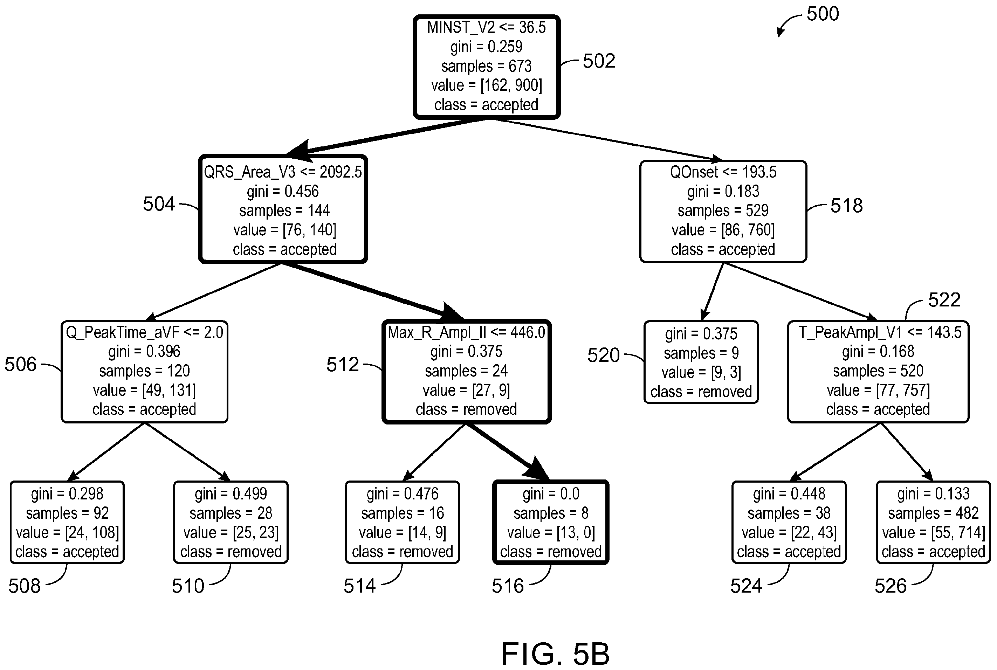

FIGS. 5A and 5B are diagrams 500 of an example AI model for extracting criteria for determining a diagnosis by a rule-based ECG analysis model. The diagrams 500 may correspond to a particular diagnosis of left bundle branch block (LBBB). The AI model 160 may include a root node 502, an intermediate node 504, an intermediate node 506, a leaf node 508, a leaf node 510, an intermediate node 512, a leaf node 514, a leaf node 516, an intermediate node 518, a leaf node 520, an intermediate node 522, a leaf node 524, and a leaf node 526.

The root node 502 corresponds to a comparison of a feature ("MINST _V2") and a threshold ("36.5"). The Gini index of the root node 502 is 0.259. From the training data, 900 data points included an acceptance, by a physician, of the diagnosis of LBBB determined by the rule-based ECG analysis model 140 where the feature was less than or equal to the threshold. Further, from the training data, 162 data points included a removal, by a physician, of the diagnosis of LBBB determined by the rule-based ECG analysis model 140 where the feature was less than or equal to the threshold. The root node 502 includes a classification target that identifies that the diagnosis of LBBB should be accepted in the event that the feature is less than or equal to the threshold.

The intermediate node 504 corresponds to a comparison of a feature ("QRS_Area_V3") and a threshold ("-2092.5"). The Gini index of the intermediate node 504 is 0.456. From the training data, 140 data points included an acceptance, by a physician, of the diagnosis of LBBB determined by the rule-based ECG analysis model 140 where the feature was less than or equal to the threshold. Further, from the training data, 76 data points included a removal, by a physician, of the diagnosis of LBBB determined by the rule-based ECG analysis model 140 where the feature was less than or equal to the threshold. The intermediate node 504 includes a classification target that identifies that the diagnosis of LBBB should be accepted in the event that the feature is less than or equal to the threshold.

The intermediate node 506 corresponds to a comparison of a feature ("Q_PeakTime_aVF") and a threshold ("2.0"). The Gini index of the intermediate node 506 is 0.396. From the training data, 131 data points included an acceptance, by a physician, of the diagnosis of LBBB determined by the rule-based ECG analysis model 140 where the feature was less than or equal to the threshold. Further, from the training data, 49 data points included a removal, by a physician, of the diagnosis of LBBB determined by the rule-based ECG analysis model 140 where the feature was less than or equal to the threshold. The intermediate node 506 includes a classification target that identifies that the diagnosis of LBBB should be accepted in the event that the feature is less than or equal to the threshold.

The leaf node 508 has a Gini index of 0.298. From the training data, 108 data points included an acceptance, by a physician, of the diagnosis of LBBB determined by the rule-based ECG analysis model 140 where the criteria of the root node 502, the intermediate node 504, and the intermediate node 506 were satisfied. Further, from the training data, 24 data points included a removal, by a physician, of the diagnosis of LBBB determined by the rule-based ECG analysis model 140 where the criteria of the root node 502, the intermediate node 504, and the intermediate node 506 were satisfied. The leaf node 508 includes a classification target that identifies that the diagnosis of LBBB should be accepted in the event that the criteria of the root node 502, the intermediate node 504, and the intermediate node 506 are satisfied.

The leaf node 510 has a Gini index of 0.499. From the training data, 23 data points included an acceptance, by a physician, of the diagnosis of LBBB determined by the rule-based ECG analysis model 140 where at least one of the criteria of the root node 502, the intermediate node 504, and the intermediate node 506 was not satisfied. Further, from the training data, 25 data points included a removal, by a physician, of the diagnosis of LBBB determined by the rule-based ECG analysis model 140 where at least one of the criteria of the root node 502, the intermediate node 504, and the intermediate node 506 was not satisfied. The leaf node 510 includes a classification target that identifies that the diagnosis of LBBB should be removed in the event that at least one of the criteria of the root node 502, the intermediate node 504, and the intermediate node 506 is not satisfied.

The intermediate node 512 corresponds to a comparison of a feature ("Max_R_Ampl_II") and a threshold ("446.0"). The Gini index of the intermediate node 512 is 0.375. From the training data, 9 data points included an acceptance, by a physician, of the diagnosis of LBBB determined by the rule-based ECG analysis model 140 where the feature was less than or equal to the threshold. Further, from the training data, 27 data points included a removal, by a physician, of the diagnosis of LBBB determined by the rule-based ECG analysis model 140 where the feature was less than or equal to the threshold. The intermediate node 512 includes a classification target that identifies that the diagnosis of LBBB should be removed in the event that the feature is less than or equal to the threshold.

The leaf node 514 has a Gini index of 0.476. From the training data, 9 data points included an acceptance, by a physician, of the diagnosis of LBBB determined by the rule-based ECG analysis model 140 where at least one of the criteria of the root node 502, the intermediate node 504, and the intermediate node 512 was not satisfied. Further, from the training data, 14 data points included a removal, by a physician, of the diagnosis of LBBB determined by the rule-based ECG analysis model 140 where at least one of the criteria of the root node 502, the intermediate node 504, and the intermediate node 512 was not satisfied. The leaf node 514 includes a classification target that identifies that the diagnosis of LBBB should be removed in the event that at least one of the criteria of the root node 502, the intermediate node 504, and the intermediate node 512 is not satisfied.

The leaf node 516 has a Gini index of 0.0. From the training data, 0 data points included an acceptance, by a physician, of the diagnosis of LBBB determined by the rule-based ECG analysis model 140 where the criteria of the root node 502, the intermediate node 504, and the intermediate node 512 were satisfied. Further, from the training data, 13 data points included a removal, by a physician, of the diagnosis of LBBB determined by the rule-based ECG analysis model 140 where at least one of the criteria of the root node 502, the intermediate node 504, and the intermediate node 512 were not satisfied. The leaf node 514 includes a classification target that identifies that the diagnosis of LBBB should be removed in the event that the criteria of the root node 502, the intermediate node 504, and the intermediate node 512 are not satisfied.

The intermediate node 518 corresponds to a comparison of a feature ("QOnset") and a threshold ("193.5"). The Gini index of the intermediate node 518 is 0.183. From the training data, 760 data points included an acceptance, by a physician, of the diagnosis of LBBB determined by the rule-based ECG analysis model 140 where the feature was less than or equal to the threshold. Further, from the training data, 86 data points included a removal, by a physician, of the diagnosis of LBBB determined by the rule-based ECG analysis model 140 where the feature was less than or equal to the threshold. The intermediate node 518 includes a classification target that identifies that the diagnosis of LBBB should be accepted in the event that the feature is less than or equal to the threshold.

The leaf node 520 has a Gini index of 0.375. From the training data, 3 data points included an acceptance, by a physician, of the diagnosis of LBBB determined by the rule-based ECG analysis model 140 where at least one of the criteria of the root node 502 and the intermediate node 518 was not satisfied. Further, from the training data, 9 data points included a removal, by a physician, of the diagnosis of LBBB determined by the rule-based ECG analysis model 140 where at least one of the criteria of the root node 502 and the intermediate node 512 was not satisfied. The leaf node 514 includes a classification target that identifies that the diagnosis of LBBB should be removed in the event that at least one of the criteria of the root node 502 and the intermediate node 518 is not satisfied.

The intermediate node 522 corresponds to a comparison of a feature ("T_PeakAmple_V1") and a threshold ("143.5"). The Gini index of the intermediate node 522 is 0.168. From the training data, 757 data points included an acceptance, by a physician, of the diagnosis of LBBB determined by the rule-based ECG analysis model 140 where the feature was less than or equal to the threshold. Further, from the training data, 77 data points included a removal, by a physician, of the diagnosis of LBBB determined by the rule-based ECG analysis model 140 where the feature was less than or equal to the threshold. The intermediate node 522 includes a classification target that identifies that the diagnosis of LBBB should be accepted in the event that the feature is less than or equal to the threshold.

The leaf node 524 has a Gini index of 0.448. From the training data, 43 data points included an acceptance, by a physician, of the diagnosis of LBBB determined by the rule-based ECG analysis model 140 where at least one of the criteria of the root node 502, the intermediate node 518, and the intermediate node 522 was not satisfied. Further, from the training data, 22 data points included a removal, by a physician, of the diagnosis of LBBB determined by the rule-based ECG analysis model 140 where at least one of the criteria of the root node 502, the intermediate node 518, and the intermediate node 522 was not satisfied. The leaf node 524 includes a classification target that identifies that the diagnosis of LBBB should be accepted in the event that at least one of the criteria of the root node 502, the intermediate node 518, and the intermediate node 522 was not satisfied.

The leaf node 526 has a Gini index of 0.133. From the training data, 714 data points included an acceptance, by a physician, of the diagnosis of LBBB determined by the rule-based ECG analysis model 140 where the root node 502, the intermediate node 518, and the intermediate node 522 were satisfied. Further, from the training data, 55 data points included a removal, by a physician, of the diagnosis of LBBB determined by the rule-based ECG analysis model 140 where the root node 502, the intermediate node 518, and the intermediate node 522 were satisfied. The leaf node 526 includes a classification target that identifies that the diagnosis of LBBB should be accepted in the event that the root node 502, the intermediate node 518, and the intermediate node 522 are satisfied.

As shown in FIG. 5B, the platform 150 may determine a decision branch of the AI model 160 that includes one or more nodes corresponding to criteria having an attribute selection measure that satisfies a threshold. In this case, the decision branch may include the root node 502, the intermediate node 504, the intermediate node 512, and the leaf node 516. The leaf node includes an attribute selection measure (e.g., Gini index) that is less than a threshold. Accordingly, the platform 150 may extract the criteria corresponding to the root node 502, the intermediate node 504, the intermediate node 512, and the leaf node 516, and generate a rule for the rule-based ECG analysis model 140 based on the criteria.

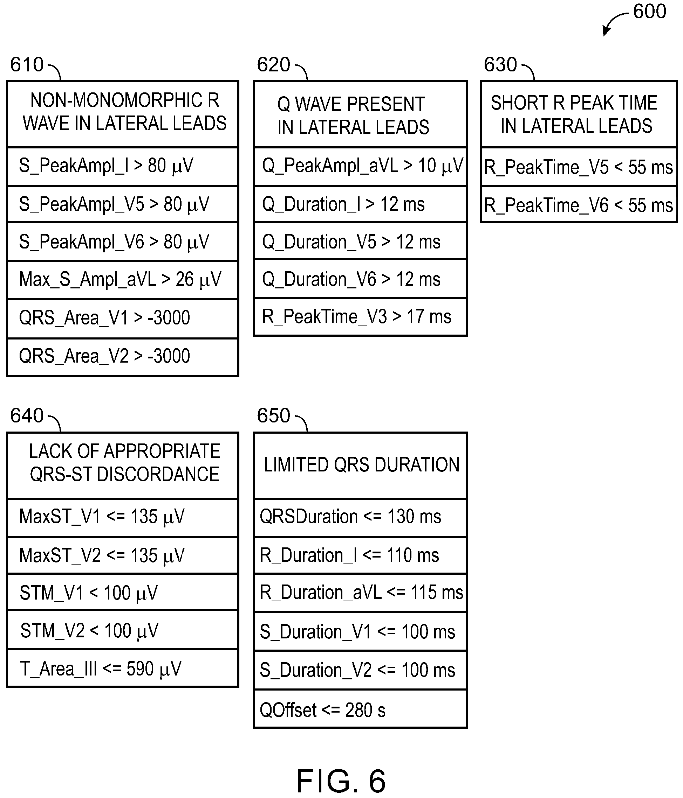

FIG. 6 is a diagram 600 of example criteria for determining a diagnosis by a rule-based ECG analysis model. As shown in FIG. 6, the platform 150 may generate a first group 610 of criteria, a second ground 620 of criteria, a third group 630 of criteria, a fourth group of criteria 640, and a fifth group 650 of criteria. The platform 150 may generate the groups of criteria based on extracting the criteria from respective decision branches of the AI model 160. The platform 150 may group the criteria based on some commonality or similarity of the criteria, and generate the groups based on grouping the criteria. The platform 150 may then generate one or more rules using the criteria in the groups of criteria.

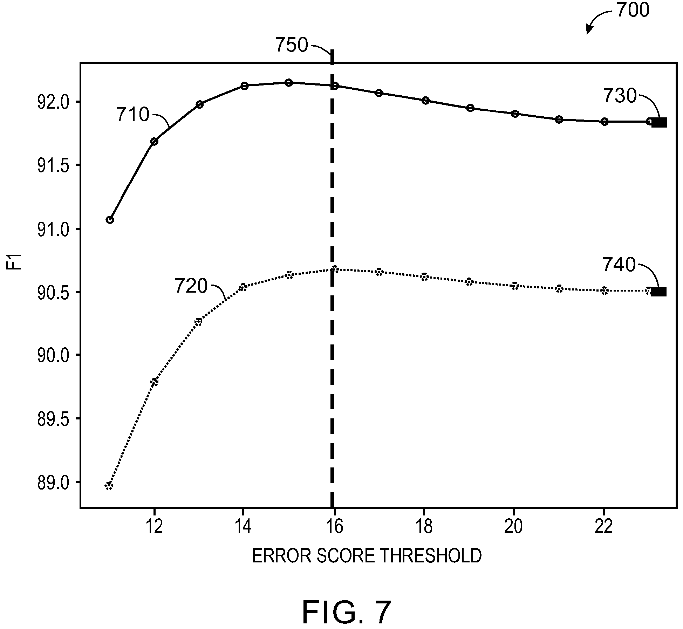

FIG. 7 is a diagram 700 of an example graph of an F1 score and an error score threshold. As shown in FIG. 7, the graph may include an x-axis corresponding to an error score threshold, a y-axis corresponding to an F1 score, a first curve 710, and a second curve 720. The first curve 710 may include a baseline point 730 corresponding to a maximum error score threshold. The second curve 720 may have a baseline point 740 corresponding to a maximum error score threshold. The platform 150 may set the threshold 750 using an optimization technique to improve the flexibility of the rule-based ECG analysis model 140.

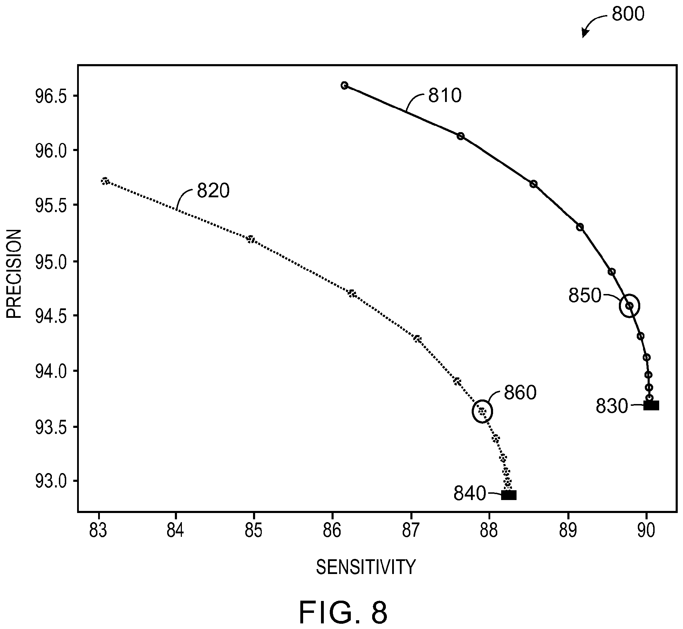

FIG. 8 is a diagram 800 of an example graph of a precision and a sensitivity of the rule-based ECG analysis model. As shown in FIG. 7, the graph may include an x-axis corresponding to a sensitivity value, a y-axis corresponding to a precision value, a first curve 810, and a second curve 820. The first curve 810 may include a baseline point 830 corresponding to a maximum sensitivity, and the second curve 820 may have a baseline point 840 corresponding to a maximum sensitivity. The first curve 810 may include a point 850 corresponding to the set error score threshold, and the second curve 820 may include a point 860 corresponding to the set error score threshold.

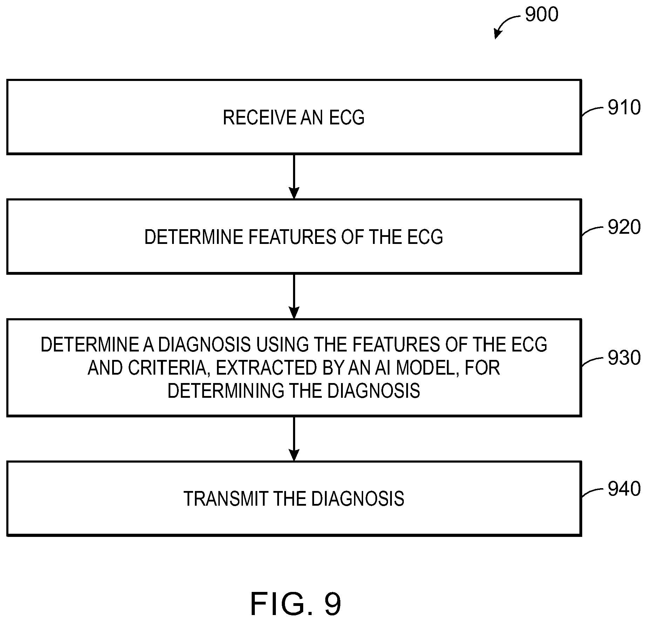

FIG. 9 is a flowchart of an example process 900 for determining a diagnosis using features of an ECG and criteria, extracted by an AI model, for determining the diagnosis.

As shown in FIG. 9, the process 900 may include receiving an ECG (operation 910). For example, the ECG analysis device 130 may receive an ECG acquired by the ECG device 110. The ECG analysis device 130 may receive the ECG from the ECG device 110, from the database 180, from the user device 170, or the like. The ECG analysis device 130 may receive the ECG based on the ECG being acquired by the ECG device 110, based on an instruction from the user device 170, or the like. The ECG analysis device 130 may receive the ECG, and input the ECG to the rule-based ECG analysis model 140.

As further shown in FIG. 9, the process 900 may include determining features of the ECG (operation 920). For example, the ECG analysis device 130 may determine features of the ECG using the rule-based ECG analysis model 140. The features of the ECG may include an amplitude of a P wave, a duration of the P wave, an amplitude of a Q wave, a duration of the Q wave, an amplitude of an R wave, a duration of the R wave, a PR interval, an amplitude of an S wave, a duration of the S wave, a duration of the QRS complex, an amplitude of a T wave, a duration of the T wave, a QT interval, or the like.

As further shown in FIG. 9, the process 900 may include determining a diagnosis using the features of the ECG and criteria, extracted by an AI model, for determining the diagnosis (operation 930). For example, the ECG analysis device 130 may determine a diagnosis using the features of the ECG and criteria extracted by the AI model 160. The diagnosis may include atrial-paced rhythm, ventricular-paced rhythm, atrial flutter, ectopic atrial tachycardia, sinus bradycardia, sinus tachycardia, junctional bradycardia, atrial fibrillation, left bundle branch block, septal infarct, or the like. The criteria may be extracted by the AI model 160, and may be criteria that are associated with an attribute selection measure that satisfies a threshold. For example, the attribute selection measure may be information gain, a gain ratio, a Gini index, or the like.

According to an embodiment, the ECG analysis device 130 may determine an overall score based on the particular criterion, of the criteria, that are satisfied. The ECG analysis device 130 may compare the overall score to a score threshold, and determine a diagnosis based on comparing the overall score to the score threshold. For example, if the overall score is greater than or equal to the score threshold, then the ECG analysis device 130 may determine a particular diagnosis. Alternatively, if the overall score is less than the score threshold, then the ECG analysis device 130 may not determine the particular diagnosis.

As further shown in FIG. 9, the process 900 may include transmitting the diagnosis (operation 940). For example, the ECG analysis device 130 may transmit the diagnosis as a diagnostic interpretation result to the user device 170 for display. Alternatively, the ECG analysis device 130 may transmit the diagnosis as a diagnostic interpretation result to a display of the ECG analysis device 130 for display.

Although FIG. 9 depicts particular operations and a particular sequence of operations, it should be understood that other embodiments may include different operations, more operations, fewer operations, or differently arranged operations than as shown in FIG. 9.

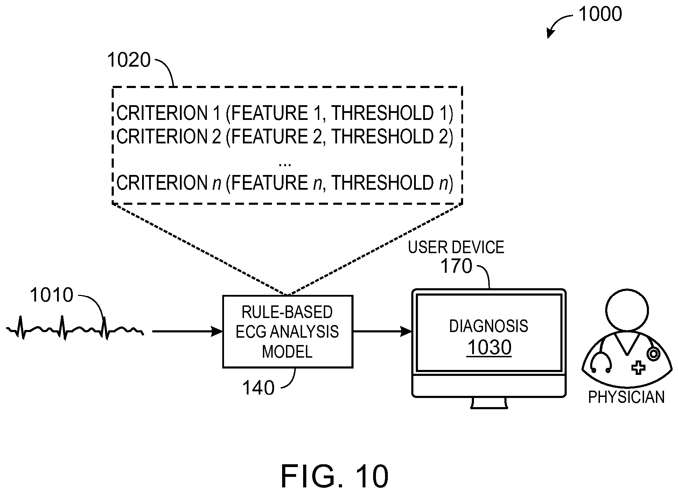

FIG. 10 is a diagram of an example process 1000 for determining a diagnosis using features of an ECG and criteria, extracted by an AI model, for determining the diagnosis. As shown in FIG. 10 by reference number 1010, the rule-based ECG analysis model 140 may receive an ECG. As shown by reference number 1020, the rule-based ECG analysis model 140 may determines a set of features (e.g., "feature 1," "feature 2," . . . "feature n"), and compare the set of features to respective thresholds (e.g., "threshold 1," "threshold 2," . . . "threshold *n*") of corresponding criteria (e.g., "criterion 1," "criterion 2," . . . "criterion n"). As shown by reference number 1030, the rule-based ECG analysis model 140 may determine a diagnosis, and transmit the diagnosis to the user device 170 for display.

Some embodiments of the present disclosure provide a system and method for extracting, using an AI model, interpretable criteria for determining a diagnosis by a rule-based ECG analysis model. Some embodiments herein may improve the performance of rule-based ECG analysis models, which may reduce the need to correct the outputs of the rule-based ECG analysis models, may improve treatment decisions by physicians, may improve outcomes for patients, or the like. Further, the extracted criteria may be fully-interpretable, which may reduce the potential for bias, may reduce uncertainty and confusion in the decision-making, and may improve the ability to integrate the extracted rules and criteria in clinical workflows.

According to an embodiment, a method may include receiving, by a rule-based electrocardiogram (ECG) model, an ECG; determining, by the rule-based ECG model, features of the ECG; determining, by the rule-based ECG model, a diagnosis using the features of the ECG and criteria extracted by an artificial intelligence (AI) model; and transmitting the diagnosis.

According to an embodiment, a device may include a memory configured to store instructions; and one or more processors configured to: receive, by a rule-based electrocardiogram (ECG) model of the device, an ECG; determine, by the rule-based ECG model of the device, features of the ECG; determine, by the rule-based ECG model of the device, a diagnosis using the features of the ECG and criteria extracted by an artificial intelligence (AI) model; and transmit the diagnosis.

According to an embodiment, a non-transitory computer-readable medium may store instructions that, when executed by one or more processors of a device, cause the one or more processors to: receive, by a rule-based electrocardiogram (ECG) model of the device, an ECG; determine, by the rule-based ECG model of the device, features of the ECG; determine, by the rule-based ECG model of the device, a diagnosis using the features of the ECG and criteria extracted by an artificial intelligence (AI) model; and transmit the diagnosis.

According to an embodiment, a method may include receiving training data for training an AI model to extract criteria for determining a diagnosis by a rule-based ECG analysis model; training the AI model based on the training data; extracting, using the AI model, the criteria for determining the diagnosis by the rule-based ECG analysis model; and updating the rule-based ECG analysis model to determine the diagnosis using the criteria.

According to an embodiment, a device may include a memory configured to store instructions; and one or more processors configured to: receive training data for training an AI model to extract criteria for determining a diagnosis by a rule-based ECG analysis model; train the AI model based on the training data; extract, using the AI model, the criteria for determining the diagnosis by the rule-based ECG analysis model; and update the rule-based ECG analysis model to determine the diagnosis using the criteria.

According to an embodiment, a non-transitory computer-readable medium may store instructions that, when executed by one or more processors of a device, cause the one or more processors to: receive training data for training an AI model to extract criteria for determining a diagnosis by a rule-based ECG analysis model; train the AI model based on the training data; extract, using the AI model, the criteria for determining the diagnosis by the rule-based ECG analysis model; and update the rule-based ECG analysis model to determine the diagnosis using the criteria.

Embodiments of the present disclosure shown in the drawings and described above are example embodiments only and are not intended to limit the scope of the appended claims, including any equivalents as included within the scope of the claims. Various modifications are possible and will be readily apparent to the skilled person in the art. It is intended that any combination of non-mutually exclusive features described herein are within the scope of the present invention. That is, features of the described embodiments can be combined with any appropriate aspect described above and optional features of any one aspect can be combined with any other appropriate aspect. Similarly, features set forth in dependent claims can be combined with non-mutually exclusive features of other dependent claims, particularly where the dependent claims depend on the same independent claim. Single claim dependencies may have been used as practice in some jurisdictions require them, but this should not be taken to mean that the features in the dependent claims are mutually exclusive.

## Claims

1. A method comprising:
receiving, by a rule-based electrocardiogram (ECG) model, an ECG;
determining, by the rule-based ECG model, features of the ECG;
determining, by the rule-based ECG model, a diagnosis using the features of the ECG and criteria extracted by an artificial intelligence (AI) model; and
transmitting the diagnosis.

2. The method of claim 1, wherein the AI model is a decision tree.

3. The method of claim 2, wherein the criteria were extracted by the AI model based on a decision branch of the AI model.

4. The method of claim 3, wherein the criteria were extracted by the AI model based on the decision branch including an attribute selection measure that satisfies a threshold.

5. The method of claim 4, wherein the attribute selection measure includes an information gain, a gain ratio, or a Gini index.

6. The method of claim 1, further comprising:
determining a score associated with the diagnosis based on respective criterion, of the criteria, being satisfied.

7. The method of claim 1, wherein the criteria include respective features of the ECG and respective thresholds for the features.

8. A device comprising:
a memory configured to store instructions; and
one or more processors configured to:
receive, by a rule-based electrocardiogram (ECG) model of the device, an ECG;
determine, by the rule-based ECG model of the device, features of the ECG;
determine, by the rule-based ECG model of the device, a diagnosis using the features of the ECG and criteria extracted by an artificial intelligence (AI) model; and
transmit the diagnosis.

9. The device of claim 8, wherein the AI model is a decision tree.

10. The device of claim 9, wherein the criteria were extracted by the AI model based on a decision branch of the AI model.

11. The device of claim 10, wherein the criteria were extracted by the AI model based on the decision branch including an attribute selection measure that satisfies a threshold.

12. The device of claim 11, wherein the attribute selection measure includes an information gain, a gain ratio, or a Gini index.

13. The device of claim 8, wherein the one or more processors are further configured to:
determine a score associated with the diagnosis based on respective criterion, of the criteria, being satisfied.

14. The device of claim 8, wherein the criteria include respective features of the ECG and respective thresholds for the features.
